# FASCICULE DE BREVET EUROPEEN

(11) **EP 3 313 462 B1**
(45) Date de publication et mention de la délivrance du brevet: **27.11.2019**
(21) Numéro de dépôt: 16733389.7
(22) Date de dépôt: 22.06.2016
(51) Int. Cl.: A61L 27/10, A61L 27/56

(54) **IMPLANT DE SUBSTITUTION DU STERNUM**
STERNUMERSATZIMPLANTAT
STERNUM REPLACEMENT IMPLANT

(30) Priorité: 23.06.2015 FR 1555761
(43) Date de publication de la demande: 02.05.2018
(73) Titulaire: I.Ceram, 87280 Limoges (FR)
(72) Inventeur: SETTON, Daniel, 87280 Limoges (FR); FIORENZA, Fabrice, 87280 Limoges (FR); BERTIN, François, 87280 Limoges (FR); STURTZ, Frank, 87280 Limoges (FR); DENES, Eric, 87280 Limoges (FR); DONNEZ, Delphine, 87280 Limoges (FR)
(74) Mandataire: August Debouzy
(86) Numéro de dépôt international: PCT/EP2016/064482
(87) Numéro de publication internationale: WO 2016/207255

(56) Documents cités:
- FR-A1- 2 823 674
- Milisavljevic et al.: "Sternum Resection and Chest Wall Reconstruction with Metaacrilate Implant in Tuberculosis", , 6 juillet 2012 (2012-07-06), XP055263856, Extrait de l'Internet: URL:http://www.ncbi.nlm.nih.gov/pmc/articl es/PMC3693342/ [extrait le 2016-04-08]
- MILOS STOJKOVIC ET AL: "Reverse modeling and solid free-form fabrication of sternum implant", AUSTRALASIAN PHYSICAL AND ENGINEERING SCIENCES IN MEDICINE., vol. 33, no. 3, 7 septembre 2010 (2010-09-07), pages 243-250, XP055264212, AU ISSN: 0158-9938, DOI: 10.1007/s13246-010-0029-1
- POLI E, ET AL.: "Does low hydroxyl group surface density explain less bacterial adhesion on porous alumina?", ORTHOPAEDICS & TRAUMATOLOGY: SURGERY & RESEARCH, vol. 105, 2019, pages 473-477,
- GRISTINA ET AL.: "Comparative in vitro Antibiotic resistance of Surface-colonizing Coagulase-Negative Staphylococci", ANTIMICROBIAL AGENTS AND CHEMOTHERAPY, vol. 33, no. 6, 2019, pages 813-819,

## Description

La présente invention a pour objet un implant de substitution du sternum.

Dans le cas de lésions uniques ou d'infections graves, une sternectomie totale peut être pratiquée. C'est le cas des cancers du sternum radio-induits (et des métastases osseuses sur sternum) et de la médiastine post opératoire (MPO), qui se produit suite à une complication postopératoire au niveau du sternum lors d'interventions cardiaques.

On cherche donc des implants de substitution du sternum.

Jusqu'à aujourd'hui, deux solutions s'offrent au chirurgien, notamment cardio-vasculaire ou orthopédiste. On peut remplacer le sternum par une prothèse en titane, mais elle présente un risque d'infection élevé et provoque des artefacts nuisant à la réalisation de radiographies, tout en présentant un poids psychologique important pour le patient. On peut aussi procéder à la construction durant l'opération d'une pièce sur-mesure en ciment osseux (PMMA) avec l'ajout de fixations métalliques, mais là encore avec un risque infectieux et un allongement de la durée de l'intervention, une difficulté d'accrochage due à l'absence de perçage et une chaleur dégagée au moment de la mise en forme avec des risques de brûlure.

Par ailleurs, on recherche une ostéointégration secondaire de l'implant dans un délai compatible avec les exigences physiologiques.

Milisavljevic et al.: "Sternum Resection and Chest Wall Reconstruction with Metaacrilate Implant in Tuberculosis", 6 juillet 2012 ,Extrait de l'Internet:URL:http:// www.ncbi.nlm.nih.gov/pmc/articles/PMC3693342/ [extrait le 2016-04-08] et MILOS STOJKOVIC ET AL: "Reverse modeling and solid free-form fabrication of sternum implant",AUSTRALASIAN PHYSICAL AND ENGINEERING SCIENCES IN MEDICINE., vol. 33, no. 3, 7 septembre 2010, pages 243-250, ,AU ISSN: 0158-9938, DOI:10.1007/s13246-010-00291 décrivent un implant de substitution du sternum à base de polyméthacrylate de méthyle.

L'invention a donc pour objet un implant de substitution du sternum à base de céramique d'alumine.

La céramique, à base d'alumine Al₂O₃, est avantageusement poreuse. Cette céramique alumine est connue en soi mais elle peut être utilisée dopée par certains autres matériaux type Zircone.

La porosité (ouverte et interconnectée) de cette céramique peut notamment être comprise entre 40 et 80%, de préférence entre 60 et 70%, avantageusement environ 65%

La taille des pores est typiquement de 200 à 600 µm, de préférence 400 µm.

La porosité/taille des pores est mesurée par porosimétrie mercure. La porosité est définie par la différence entre le volume occupé par les pores sur le volume total, le volume total étant la somme du volume des pores et de l'alumine. La masse d'alumine étant définie par le volume et la masse volumique, en pesant l'échantillon et en connaissant son volume total, on peut déterminer par différence le volume de pores et donc la porosité (ouverte).

L'alumine poreuse, matériau constitutif de l'implant selon l'invention, permet une ostéointégration secondaire de l'implant à environ 3 mois.

La résistance mécanique à la compression est avantageusement comprise entre 20 et 60MPa, avantageusement supérieure à 40MPa.

On peut utiliser généralement tout procédé connu de préparation d'alumine poreuse, notamment par imprégnation d'une mousse, préfrittage à une température supérieure à 1200°C, surimprégnation par une barbotine, et frittage à une température supérieure à 1600°C.

On peut notamment utiliser un procédé comprenant les étapes suivantes :
- (A) fourniture d'un matériau porogène (type mousse, par exemple mousse polyuréthane, servant à régler notamment la porosité et la taille des pores) et imprégnation du matériau porogène par une suspension de particules céramiques alumine (barbotine d'alumine) éventuellement en mélange avec divers additifs organiques tels que liants, plastifiants et dispersants ;
- (B) séchage en étuve ;
- (C) traitement thermique à basse température (inférieure à 700°C) pour éliminer la mousse et des constituants organiques de la suspension ; puis
- (D) frittage à une température supérieure à 1500°C.

On peut utiliser avantageusement le procédé décrit dans la demande de brevet FR2823674.

Notamment, on peut préparer la matrice céramique de l'invention en mettant en œuvre le procédé qui y est décrit. Dans le mode de réalisation préféré, après la mise en œuvre des deux premières phases telles que décrites ci-dessus (phases A, B), la pièce céramique poreuse est pré-frittée à une température supérieure à 1200°C, ce qui lui confère une cohésion supérieure (phase C'). Le cycle se poursuit par un nouveau trempage de la pièce dans une autre suspension de particules céramiques (phase E). La viscosité de cette suspension concentrée est contrôlée grâce à divers auxiliaires organiques (liants, plastifiants, dispersants), pour être adaptée à une imprégnation homogène de la pièce poreuse préfrittée. Après un nouveau séchage en étuve (phase B') et pyrolyse des auxiliaires organiques de la suspension (phase C), la pièce céramique est enfin frittée à une température supérieure à 1600°C suivant un cycle adapté (phase D').

Ce procédé de sur-imprégnation renforce les propriétés mécaniques de la céramique frittée et multiplie sa résistance par un coefficient 2, notamment la contrainte à la rupture en compression.

Une telle céramique est disponible chez le déposant, sous la référence Ceramil®.

On peut donner la forme souhaitée à la matrice par un usinage ou par une conformation directement lors du frittage.

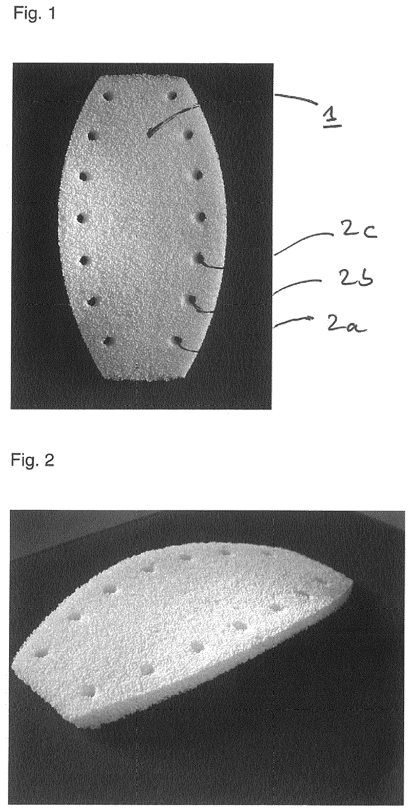

La figure 1 est un vue de dessus de l'implant (1) selon l'invention. L'implant présente des perçages latéraux (2a, 2b, 2c), de diamètre par exemple entre 0.8 et 4mm. On peut procéder par usinage, notamment par ultra-sons, pour réaliser les perçages. L'implant munis des perçages peut donc ensuite être suturé aux cartilages costaux. L'implant offre ainsi aux chirurgiens une praticité supplémentaire grâce aux perçages réalisés qui permettent un ancrage simplifié de l'implant.
La figure 2 est une autre vue de l'implant (2) selon l'invention. L'implant est sous forme d'une plaque ovoïde incurvée, qui présente une géométrie anatomique. Ceci permet un comblement de la zone réséquée en respectant la physiologie de la cage thoracique.

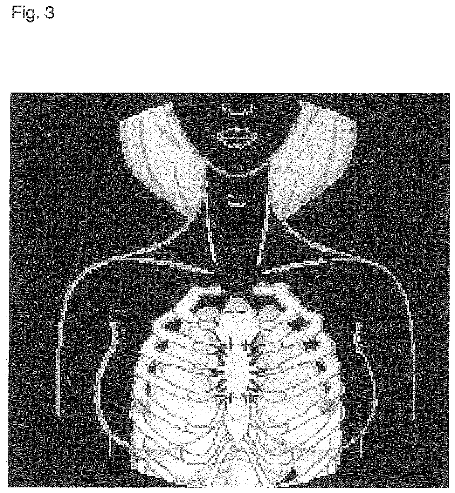

Après avoir effectué des repérages anatomiques, le chirurgien pratique une incision cutanée comprenant le trajet et le point d'entrée de la biopsie, sectionne les côtes et pratique une exérèse puis une ablation monobloc de la tumeur. Le chirurgien met en place l'implant, effectue les sutures nécessaires et le recouvre avec un lambeau de grand pectoral. L'implant en position est représenté à la figure 3.

Le sternum en céramique selon l'invention offre une biocompatibilité totale permettant une intégration osseuse durable. La non-utilisation de pièces métalliques permet de réaliser des radiographies sans artefact et ainsi de bénéficier d'une imagerie de qualité afin d'améliorer le suivi clinique. L'utilisation de l'implant selon l'invention permet également un gain de temps opératoire, ce qui réduit le risque d'infection, les études indiquant que l'allongement d'une heure d'une opération multiplie par deux le risque infectieux.

L'impact global pour le patient est également diminué. En effet, l'intégration « naturelle » de l'implant selon l'invention limite les effets psychologiques d'une telle opération. L'ostéointégration facilite l'acceptation de l'implant au sein de l'organisme et confère une plasticité au thorax après opération.

Le sternum en céramique permet également d'assurer une reproductibilité de l'opération ; selon un mode de réalisation le sternum selon l'invention est disponible sous forme d'une gamme d'implants, avec par exemple 3 tailles d'implants.

## Revendications

1. Implant de substitution (1) du sternum comprenant une céramique d'alumine poreuse.

2. Implant (1) selon la revendication 1, **caractérisé en ce que** la céramique d'alumine présente une porosité en volume de 45 à 75% et une taille de pores de 200 à 600µm.

3. Implant (1) selon la revendication 1 ou 2, **caractérisé en ce que** la céramique d'alumine est obtenue par imprégnation d'une mousse, préfrittage à une température supérieure à 1200°C, surimprégnation par une barbotine, et frittage à une température supérieure à 1600°C.

4. Implant (1) selon l'une des revendications 1 à 3, sous forme d'une plaque ovoïde incurvée.

5. Implant (1) selon l'une des revendications 1 à 4, comprenant des perçages latéraux (2a, 2b, 2c) pour suture aux cartilages costaux.

## Patentansprüche

1. Sternumersatzimplantat (1), umfassend eine poröse Aluminium-Keramik.

2. Implantat (1) nach Anspruch 1, **dadurch gekennzeichnet, dass** die Aluminium-Keramik eine Porosität im Volumen von 45 bis 75 % und eine Porengröße von 200 bis 600 µm aufweist.

3. Implantat (1) nach Anspruch 1 oder 2, **dadurch gekennzeichnet, dass** die Aluminiumkeramik durch Imprägnierung eines Schaums, Vorsinterung bei einer Temperatur von mehr als 1200 °C, Überimprägnierung durch einen Schlicker und Sinterung bei einer Temperatur von mehr als 1600 °C erhalten wird.

4. Implantat (1) nach einem der Ansprüche 1 bis 3 in Form einer gekrümmten ovoiden Platte.

5. Implantat (1) nach einem der Ansprüche 1 bis 4, umfassend seitliche Bohrungen (2a, 2b, 2c) zur Naht an den Rippenknorpeln.

## Claims

1. Sternum replacement implant (1) comprising a porous alumina ceramic.

2. Implant (1) according to claim 1, **characterised in that** the alumina ceramic has a porosity by volume of 45 to 75% and a pore size of 200 to 600µm.

3. Implant (1) according to claim 1 or 2, **characterised in that** the alumina ceramic is obtained by impregnation of a foam, pre-sintering at a temperature greater than 1200°C, superimpregnation with a slip, and sintering at a temperature greater than 1600°C.

4. Implant (1) according to one of claims 1 to 3, in the form of a curved ovoid sheet.

5. Implant (1) according to one of claims 1 to 4, comprising lateral holes (2a, 2b, 2c) for suturing to the costal cartilages.
